# EUROPÄISCHE PATENTSCHRIFT

(11) **EP 0 799 578 B1**
(45) Veröffentlichungstag und Bekanntmachung des Hinweises auf die Patenterteilung: **03.07.2002**
(21) Anmeldenummer: 97890056.1
(22) Anmeldetag: 24.03.1997
(51) Int. Cl.: A23L 1/172, A23L 1/302, A23L 1/304, A61K 35/78

(54) **Elektrolyt-angereicherte Keimlinge enthaltendes Kombinationspräparat**
Combined preparation containing electrolyte enriched germs
Préparation combinée comprenant des germes enrichis en électrolytes

(30) Priorität: 03.04.1996 AT 60796
(43) Veröffentlichungstag der Anmeldung: 08.10.1997
(73) Patentinhaber: Fuchs, Norbert, Mag., 5571 Mariapfarr (AT); Zelch, Norbert, Dr., 5020 Salzburg (AT); Kössler, Peter, 5571 Mariapfarr (AT); Loidl, Rupert, 5571 Mariapfarr (AT)
(72) Erfinder: Fuchs, Norbert, Mag., 5571 Mariapfarr (AT); Zelch, Norbert, Dr., 5020 Salzburg (AT); Kössler, Peter, 5571 Mariapfarr (AT); Loidl, Rupert, 5571 Mariapfarr (AT)
(74) Vertreter: Alge, Daniel, Mag. Dr. rer.nat.

(56) Entgegenhaltungen:
- EP-A- 0 124 891
- EP-A- 0 129 032
- EP-A- 0 616 810
- EP-A- 0 770 324
- DE-A- 4 017 114
- FR-A- 2 163 821
- US-A- 3 751 261
- US-A- 4 237 651

## Beschreibung

Der humane Immunstoffwechsel ist abhängig von vielerlei exogenen und endogenen Faktoren, wie in entscheidendem Maße auch von der Qualität und Quantität der zugeführten Nährstoffe.

Zahlreiche Arbeiten belegen die Beeinflußbarkeit des Immunstoffwechsels durch nutritive Faktoren. In Zusammenhang mit der gezielten Zufuhr einzelner Nährstoffe bei herabgesetzter Immunleistung wurden sowohl positive als auch negative Korrelationen gefunden, insbesondere in Zusammenhang mit der Zufuhr von β-Carotin und der Häufigkeit von Lungenkrebs bei Rauchern (National Cancer Institute: Beta Carotene and Vitamin A Halted in Lung Cancer Prevention Trial, Pressemitteilung vom 18.1.1996).

Eindeutiger dagegen sind die negativen Korrelationen zur Inzidenz von Krebs und Immunerkrankungen bei jenen Personen zu sehen, die sich nach den Grundsätzen der Vollwerternährung (also regelmäßiger Konsum von Gemüse, Obst und Vollkorngetreideprodukten, wobei isolierte oder raffinierte Produkte möglichst vermieden werden) ernähren. Im Hinblick auf die Tatsache, daß Nährstoffe in vollwertigen Lebensmitteln niemals isoliert, sondern im komplexen Verband ihrer unzähligen biologischen Vorstufen und in Anwesenheit zahlloser bioaktiver Pflanzenstoffe, die primär keine nutritiven Funktionen aufweisen, vorliegen, sind die biogenen Nährstoffe im Vergleich zu synthetischen Nährstoffen völlig differenziert zu beurteilen. Demnach dürften die stabilisierenden Effekte der Vollwerternährung auf das komplexe Zusammenspiel von Vitaminen, deren biologischen Vorstufen, organisch gebundenen Mineralstoffen und Spurenelementen sowie bioaktiven Pflanzeninhaltsstoffen (wie Flavonoiden, Tanninen, Lignanen, Phenolsäuren, Phytosterinen, Protease-Inhibitoren etc.), also Stoffen des sekundären Pflanzenstoffwechsels, zurückzuführen sein.

Im Zuge der Vorarbeiten zur vorliegenden Erfindung stellte sich heraus, daß die alleinige Zufuhr von vollwertigen Lebensmitteln bei immunsuprimierten Personen nicht ausreicht, um eine bedarfsgerechte Mikronährstoffversorgung zur Stabilisierung eines entgleisten Immunsystems zu gewährleisten. Auf der anderen Seite stellte man aber auch fest, daß die Zufuhr hoher Dosen synthetischer Vitamin- und Mineralstoffgemische ebenso nicht zum Ziel, nämlich zur ausreichenden Stützung des angegriffenen Immunsystems führen kann.

In der EP 0 129 032 A2 wird eine Präparation enthaltend Vitamin C und eine zweite Komponente, welche entweder Katalase oder eine Keimkomponente oder Extrakte von Keimkomponenten ist, offenbart. Es werden nur herkömmliche Keimlinge verwendet bzw. lediglich Komponenten oder Extrakte dieser Keimlinge, nicht aber Elektrolyt-angereicherte Keimlinge.

Gemäß der EP 0 616 810 A1 wird eine Zusammensetzung enthaltend Reiskeimlinge offenbart, welche zu pharmazeutischen Zwecken, insbesondere als Mittel gegen Krebs, verwendet werden soll. Wiederum werden herkömmliche nicht Elekrolyt-angereicherte Keimlinge eingesetzt.

In der DE 40 17 114 A1 wird ein Verfahren zur Herstellung eines Getreidekeimproduktes beschrieben, bei welchem herkömmliche Keimlinge getrocknet und durch spezielle Oberflächenbehandlung präpariert werden. Auch hier wird von völlig normalen Keimlingen ausgegangen, und Zusammensetzungen enthaltend Elektrolyt-angereicherte Keimlinge werden nicht beschrieben.

In der US-PS 4 237 651 wird eine Zugabe von chelatierten Mineralstoffen bei der Inkubation von Pflanzenkeimlingen vorgesehen. Da chelatierte Mineralstoffmischungen gar nicht in das Innere von Keimen in Samen aufgenommen und verstoffwechselt werden können, entstehen dabei auch keine Elektrolyt-angereicherten Keimlinge.

Es ist daher Aufgabe der vorliegenden Erfindung, ein neuartiges Nährstoffgemisch zur Verfügung zu stellen, welches insbesondere dazu geeignet ist, das Immunsystem, vor allem ein angegriffenes oder supprimiertes Immunsystems, zu unterstützen bzw. zu stabilisieren.

Die erfindungsgemäße Aufgabe wird gelöst durch ein Kombinations präparat, umfassend 5-90% Elektrolyt-angereicherte, als Lebensmittel verwendbare Keimlinge, erhältlich durch Keimung in einer Elektrolytlösung, wobei die Elektrolytlösung 1 mg/l oder mehr Zn-, Fe-, K- und/oder Mg-Ionen, 0,5 mg/l oder mehr Cu-, Mn-, Sr - und/oder Li-Ionen und 0,1 mg/l oder mehr Se-, Mo-, Cr-, As-, V- und/oder Co-Ionen enthält, und 5-90% zugesetzte essentielle, semiessentielle und/oder nicht essentielle Mikronährstoffe, ausgewählt aus der Gruppe bestehend aus: mehrfach ungesättigte Fettsäuren, natürlichen Carotenoidgemischen, Keimextrakten, natürlichen Anthocyangemischen, natürlichen Flavenoidgemischen, natürlichen Tocopherol- und Tocotrienolgemischen, Vitaminen und Coenzymen, essentiellen und nicht-essentiellen Aminosäuren, Mineralstoffen und Mischungen derselben.

Elektrolyt-angereicherte Keimlinge sind in der Patentschritt, EP 770 324 beschrieben und weisen einen gegenüber herkömmlichen, in Leitungswasser inkubierten Keimlingen erhöhten Elektrolytgehalt auf. Diese Elektrolyt-angereicherten Keimlinge weisen bevorzugterweise einen im Vergleich zu herkömmlich aufge keimten Samen um mindestens 10 bis 20 %, vorzugsweise um minde stens das 1,5- bis 3-fache, insbesondere um mindestens das 5- bis 10-fache, erhöhten Gehalt an einem oder mehreren Elektroly ten, vorzugsweise an Zink, Eisen, Kalium, Magnesium, Kupfer, Mangan, Strontium, Selen, Molybdän, Chrom, Arsen, Lithium, Vanadium und/oder Kobaltionen, auf.

Es hat sich gezeigt, dass derart Elektrolyt-angereicherte Keim linge nicht nur eine höhere Konzentration an Mineralstoffen aufweisen, sondern, bedingt durch den erhöhten Mineralstoff gehalt, auch ganz allgemein im Hinblick auf ihre Inhaltsstoffe verbessert sind, beispielsweise einen erhöhten Vitamingehalt aufweisen.

Diese Keimlinge werden vorzugsweise durch Keimung in einer Elek trolytlösung zwischen 10 und 50°C, vorzugsweise zwischen 20 und 30°C, während einer Zeitdauer von 12 bis 40 Stunden, vorzugswei se von etwa 60 bis 100 Stunden, erhalten, wobei die Elektrolyt lösung 1 mg/l oder mehr, vorzugsweise 10 mg/l oder mehr, ins besondere 50 mg/l oder mehr, Zink, Eisen, Kalium und/oder Magnesiumionen, 0,5 mg/l oder mehr, vorzugsweise 5 mg/l oder mehr, insbesondere 25 mg/l oder mehr, Kupfer, Mangan, Strontium und/oder Lithiumionen, 0,1 mg/l oder mehr, vorzugsweise 1 mg/l oder mehr, insbesondere 5 mg/l oder mehr, Selen, Molybdän, Chrom, Arsen, Vanadium und/oder Kobaltionen, enthält.

Keimlinge stellen an sich eine wertvolle Bereicherung der Nahrung dar, da sie beispielsweise im Vergleich zu Gemüsesorten preiswert, stets frisch, saisonunabhängig, ballaststoffreich, vitamin- und mineralstoffreich und zudem schmackhaft und gut bekömmlich sind. Bevorzugterweise werden Keimlinge von Hülsenfrüchten und Getreidesamen, wie z. B. Weizen-, Buchweizen-, Hafer-, Quinoa-, Mungobohnen-, Bockshornklee-, Rettich-, Alfalfa-, Mais-, Kürbis-, Roggen-, Gerste-, Reis-, Adzuki-Bohnen-, Erbsen-, Hirse-, Kichererbsen-, Kresse-, Leinsamen-, Linsen-, Senf-, Sesam-, Sojabohnen-, Sonnenblumen- und Amaranthkeimlinge, insbesondere Weizen-, Buchweizen- und Haferkeimlinge, im Rahmen der erfindungsgemäßen Präparation verwendet.

Wie erwähnt, konnte die Zufuhr von vollwertigen Lebensmitteln alleine bei immunsupprimierten Personen nicht zu einer ausreichenden Stabilisierung des Immunsystems führen. Möglicherweise ist dies durch den sehr individuellen Bedarf dieser Patienten an Mikronährstoffen bedingt, da überraschenderweise durch das erfindungsgemäße Kombinationspräparat eine deutliche Verbesserung des Immunstoffwechsels hervorgerufen werden kann.

Auf der anderen Seite ist die schlechte Wirkung von reinen Mikronährstoffgaben bei immunsupprimierten Personen wahrscheinlich darauf zurückzuführen, daß die erwähnten sekundären Pflanzenstoffe nicht vorhanden sind.

Unter Mikronährstoffen sind im Rahmen der vorliegenden Erfindung stoffwechselaktivierende Substanzen (im Gegensatz zu energieliefernden Substanzen) zu verstehen, die in der natürlichen Nahrung und gleichzeitig natürlicherweise im menschlichen Organismus vorhanden sind und auf deren ausreichende Zufuhr der menschliche Körper, insbesondere der durch Krankheit, Stress, etc. geschwächte Körper, angewiesen ist bzw. bei deren Zufuhr sich positive stoffwechselaktivierende Wirkungen auf den Körper ergeben, wie z.B. Vitamine, Mineralstoffe, Spurenelemente, Fettsäuren, Aminosäuren, Enzyme und sekundäre (bioaktive) Pflanzeninhaltsstoffe (siehe "Handbuch der orthomolekularen Medizin", Dietl & Ohlenschläger (1994), Haug-Verlag).

Als essentielle Mikronährstoffe werden jene angesehen, die zum Leben absolut unentbehrlich sind; semiessentielle Mikronährstoffe können gegebenenfalls durch andere Mikronährstoffe ersetzt werden oder können in gewissen pathologischen Zuständen zu essentiellen Mikronährstoffen werden; als nicht-essentielle Mikronährstoffe sind diejenigen Mikronährstoffe anzusehen, die zwar nicht unbedingt zum (Über-) Leben notwendig sind, welche jedoch eine positive, stoffwechselaktivierende Wirkung aufweisen.

Der auf Grund der der erfindungsgemäßen Kombination dieser zwei Komponenten erreichte synergetische Effekt ist daher wahrscheinlich auf das Zusammenwirken der biogenen Nährstoffanteile aus der Vollwertkomponente mit den Mikronährstoffen zurückzuführen.

Das erfindungsgemäße Kombinationspräparat enthält zusätzlich noch ein oder mehrere Gemüsekonzentrate, vorzugsweise aus Broccoli, Petersilie und/oder Blumenkohl.

Das erfindungsgemäße Präparat ist gemäß einer bevorzugten Ausführungsform aus 5 bis 90 %, vorzugsweise 20 bis 70 %, Keimlingen, aus 5 bis 90 %, vorzugsweise 20 bis 70 % Mikronährstoffen und 0 bis 90 % anderen Komponenten, vorzugsweise Gemüsekonzentraten, lebensmitteltechnischen Zusatzstoffen und/oder Hilfskomponenten zusammengesetzt. Unter lebensmitteltechnischen Zusatzstoffen oder Hilfskomponenten sind sämtliche, bei der Herstellung und Endverarbeitung bzw. verkaufsfertigen Herrichtung gängigen Zusatzstoffe zu verstehen, wobei aber nach Möglichkeit auch hier darauf zu achten ist, daß den ernährungsphysiologischen und ökologischen Gesichtspunkten der Vollwerternährung Rechnung getragen wird.

Wenn das erfindungsgemäße Kombinationspräparat als Arzneimittel zum Einsatz kommt, kann es selbstverständlich die für die jeweilige Verabreichungsform notwendigen bzw. gängigen pharmazeutischen Zusatz-, Hilfs- oder Wirkstoffe umfassen.

Gemäß einer bevorzugten Ausführungsform der vorliegenden Erfindung wird das Kombinationspräparat in Form von Kapseln, Kautabletten, Pulvermischungen oder insbesondere Kombinationen hievon und/oder in einer lebensmitteltechnisch oder arzneimitteltechnisch geeigneten Form lagerstabil abgepackt zur Verfügung gestellt. Bei der pharmazeutischen Verwendung der erfindungsgemäßen Kombinationspräparate werden diese vorzugsweise in einer oral zu verabreichenden pharmazeutischen Darreichungsform zur Verfügung gestellt.

Gleichwohl können die erfindungsgemäßen KombinationsprAparate als Zusatz mit herkömmlichen Lebensmitteln vermischt werden, was sich insbesondere bei hoch raffinierten vitamin- und mineralarmen Lebensmitteln empfiehlt.

Die Mikronährstoffkomponente im erfindungsgemäßen Kombinationspräparat ist vorzugsweise ausgewählt aus der Gruppe bestehend aus:
- mehrfach-ungesättigten Fettsäuren, vorzugsweise einem PUFA-Gemisch aus Fischöl und Borretschöl,
- natürlichen Carotenoidgemischen, vorzugsweise aus Dunaliella salina,
- Keimextrakten, vorzugsweise Weizenkeimextrakt mit Octacosanol,
- natürlichen Anthocyangemischen, vorzugsweise aus Hibiskusblüten,
- natürlichen Flavenoidgemischen, vorzugsweise aus Zitrusfrüchten, Baumharzen und Rindenextrakten,
- natürlichen Tocopherol- und Tocotrienolgemischen, vorzugsweise aus Weizenkeimen,
- Vitaminen und Coenzymen, vorzugsweise Coenzym Q10, Pyridoxol, Riboflavin, Folsäure, Biotin, Vitamin K, Vitamin B 12, Vitamin D3, Carnitin, Betain und/oder Vitamin C,
- essentiellen und nicht essentiellen Aminosäuren, vorzugsweise N-Acetylcystein, Taurin, L-Glutamin, Isoleucin, Leucin, Lysin, Methionin, Phenylalanin, Threonin, Tryptophan, Valin, Histidin, Arginin und/oder Tyrosin,
- Mineralstoffen, vorzugsweise Kieselerde, Calcium, Vanadium, Mangan, Eisen, Kalium, Zink, Kupfer, Lithium, Fluor, Germanium, Strontium, Chrom, Molybdän, Selen und/oder Jod, und
- anderen Mikronährstoffen, vorzugsweise Quercetin, α-Liponsäure, Glutathion, Aneurin, Inosit, Orotsäure, Inosin und/oder p-Aminobenzoesäure,
- oder Mischungen derselben.

Die jeweilige Konzentration der Mikronährstoffe kann vorzugsweise im Bereich der bekannten Anwendungsvorschriften dieser Stoffe liegen, sie kann aber auch je nach dem individuellen Bedarf der zu behandelnden immunsupprimierten Personen auch mehr oder weniger stark von üblichen Verabreichungskonzentrationen abweichen.

Es zeigte sich, daß mit einer zur regelmäßigen Verabreichung geeigneten täglichen maximalen Zufuhrdosis wie folgt: 78 mg β-Carotin, 10 mg Vitamin B1, 15 mg Vitamin B2, 20 mg B6, 150 mg Niacin, 30 mg Pantothensäure, 0,02 mg B12, 1,5 mg Folsäure, 1700 mg C, 0,01 mg D3, 180 mg E, 0,04 mg K, 0,3 mg Biotin, 1400 mg Kalium, 700 mg Kalzium, 550 mg Magnesium, 29 mg Eisen, 34 mg Zink, 31 mg Mangan, 8 mg Kupfer, 0,3 mg Selen, 0,2 mg Chrom, 0,2 mg Molybdän, 450 mg Natrium, 440 mg Chlorid, 770 mg Phosphor, 120 mg Coenzym Q10, 350 mg α-Liponsäure, 3 mg Lithium, 3 mg Strontium, 136 mg Flavonoide, 700 mg L-Carnitin, 250 mg Glutathion, sehr günstige, weil effektive Ergebnisse erzielt werden können. Die tägliche Mindestdosis sollte sich in einem Bereich, welcher etwa bei einem Drittel dieser empfohlenen Maximalwerte liegt, bewegen.

Wie erwähnt, ist ein wichtiger Aspekt der vorliegenden Erfindung die prophylaktische, aber insbesondere auch die therapeutische Anwendung des erfindungsgemäßen Kombinationspräparates.

Es hat sich herausgestellt, daß mit dem Kombinationspräparat gemäß der vorliegenden Erfindung immunsupprimierte Personen, insbesondere HIV-positive Patienten, welche bereits unter einer Beeinträchtigung der Funktionalität des Immunsystems leiden, effizient behandelt werden können, wobei sich durch das erfindungsgemäße Kombinationspräparat eine Verbesserung bzw. Stabilisierung des Immunsystems erzielen läßt.

Es zeigte sich, daß nicht nur die Konzentration der T-Helferzellen im Blut bzw. der Quotient aus T-Helferzellen und T-Suppressorzellen bei HIV-Positiven und AIDS-Kranken deutlich gesteigert werden kann, sondern auch ein pathologisch erhöhter Mikroglobulin- oder Neopterin-Spiegel bei der Anwendung des erfindungsgemäßen Präparates wieder gesenkt werden kann.

Die erfindungsgemäßen Präparate eignen sich aber auch für eine Reihe nicht-medizinischer Anwendungen, beispielsweise als Diätetikum oder als Nahrungsergänzung.

Die Erfindung wird durch die nachfolgenden Beispiele, auf die sie jedoch nicht beschränkt sein soll, weiter erläutert.

### Beispiele:

Ein erfindungsgemäßes Kombinationspräparat wurde drei Patienten A, B, C über einen Zeitraum von 11 bis 29 Monaten in den folgenden Tagesmengen verabreicht:

2,72 g Broccolikonzentrat, 2,6 g Petersilienkonzentrat, 2,68 g Blumenkohlkonzentrat, 15 g Weizenkeime, 5 g Weizenkeimextrakt, 15 g Lecithin, 6,4 g Haferflocken, 5 g getrocknete Aprikosen, 3 g getrocknete nährstoffangereicherte Buchweizensprossen, 3 g getrocknete nährstoffangereicherte Weizensprossen, 3,6 g getrocknete nährstoffangereicherte Hafersprossen, 6 g getrocknete Bananenfrüchte, 0,9 g L-Alanin, 0,7 g L-Arginin, 0,57 g L-Asparaginsäure, 1 g L-Glutaminsäure, 2,4 g L-Glycin, 0,06 g L-Histidin, 0,12 g L-Isoleucin, 0,3 g L-Leucin, 0,38 g L-Lysin, 0,08 g L-Methionin, 0,25 g L-Phenylalanin, 0,12 g L-Prolin, 0,28 g L-Serin, 0,15 g L-Threonin, 0,05 g L-Tyrosin, 0,25 g L-Valin, 0,006 g L-Cystin, 1 g Kalium, 500 mg Magnesium, 500 mg Natrium, 0,2 mg Molybdän, 0,5 mg Biotin, 3 mg Lithium, 3 mg Kupfer, 10 mg Zink, 50 mg Inosin, 50 mg Paraaminobenzoesäure, 5 mg Germanium, 10 mg Mangan, 50 mg Betain, 100 mg Coenzym Q10, 100 mg Taurin, 40 mg EPA, 500 mg N-Acetylcystein, 330 mg Bioflavonoide, 330 mg Anthocyane, 150 mg GLA, 100 mg L-Glutamin, 0,01 mg Vitamin D3, 0,01 mg Vitamin B12, 0,07 mg Vitamin K, 0,2 mg Iodid, 1,8 mg Fluorid, 0,2 mg Selen, 0,75 mg Folsäure, 0,2 mg Chrom, 3 mg Strontium, 15 mg Pantothensäure, 25 mg Vitamin B2, 0,4 mg Vanadium, 25 mg Vitamin B1, 2 mg Octacosanol, 50 mg Niacin, 250 mg reduziertes Glutathion, 50 mg Vitamin B6, 50 mg Orotsäure, 10 mg Eisen, 124 mg Kalzium, 200 mg Silicium, 50 mg β-Carotin. Während dieser Zeit wurde regelmäßig eine umfassende Anzahl von Parametern in diesen Patienten untersucht.

### Patient A:

Patient A ernährte sich während des Zeitraumes von der 1. bis zur 4. Untersuchung vollwertig, jedoch ohne Mikronährstoffsupplementierung; während des Zeitraumes zwischen der 4. und 5. Untersuchung ernährte sich der Patient mit dem erfindungsgemäßen Kombinationspräparat, also vollwertig mit supplementierten Mikronährstoffen; während des Zeitraumes zwischen der 5. und 7. Untersuchung schließlich ernährte sich der Patient vollwertig, nahm aber wiederum keine Mikronährstoffe zusätzlich ein. Die Ergebnisse der Untersuchungen bezüglich T-Helfer- und T-Suppressorzellen bzw. deren Verhältnis sind in der Tabelle 1 zusammengefaßt.

Die Gesamtheit der untersuchten Daten geht aus Tabelle 2 hervor.

**TABELLE 1**

| Patient A HIV-pos., männl. | 1.Untersuchung | 4.Untersuchung 9 Monate | 5.Untersuchung 12 Monate | 7.Untersuchung 24 Monate |
|---|---|---|---|---|
| T-Helfer | 540 | 250 | 710 | 392 |
| T-Suppressor | 690 | 360 | 770 | 685 |
| Ratio | 0,78 | 0,69 | 0,92 | 0,57 |

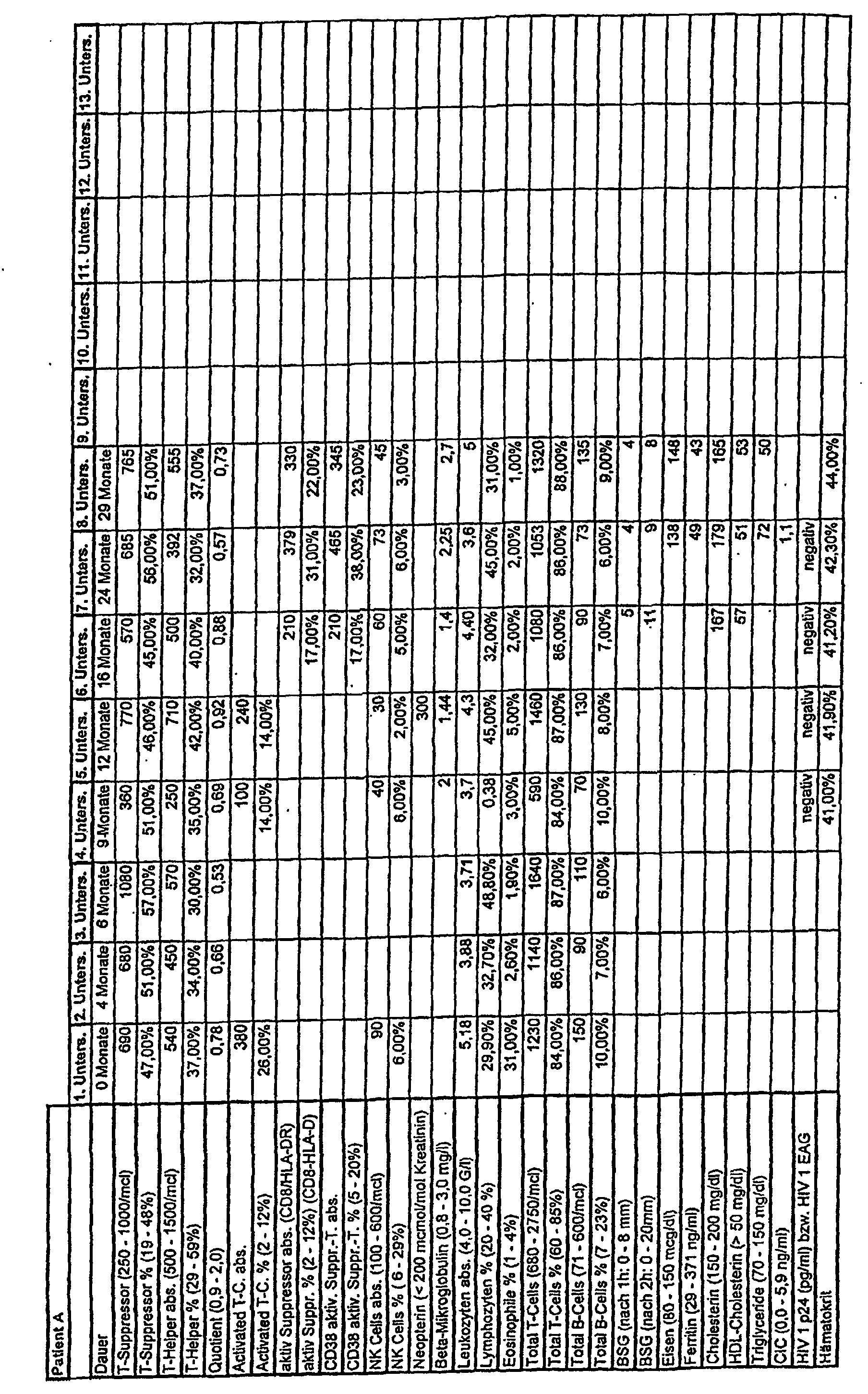

### Patientin B:

Patientin B ernährte sich während des Zeitraumes zwischen 1. und 4. Untersuchung (8 Monate) im wesentlichen vollwertig, supplementierte zusätzlich Mikronährstoffe. Die Ergebnisse der T-Helfer/T-Suppressor-Untersuchungen sind in Tabelle 3 dargestellt (Gesamtanalyse: Tabelle 4).

**TABELLE 3**

| Patientin B HIV-neg., weiblich | 1. Untersuchung | 4. Untersuchung 20 Monate |
|---|---|---|
| T-Helfer | 550 | 789 |
| T-Suppressor | 440 | 520 |
| Ratio | 1,25 | 1,52 |

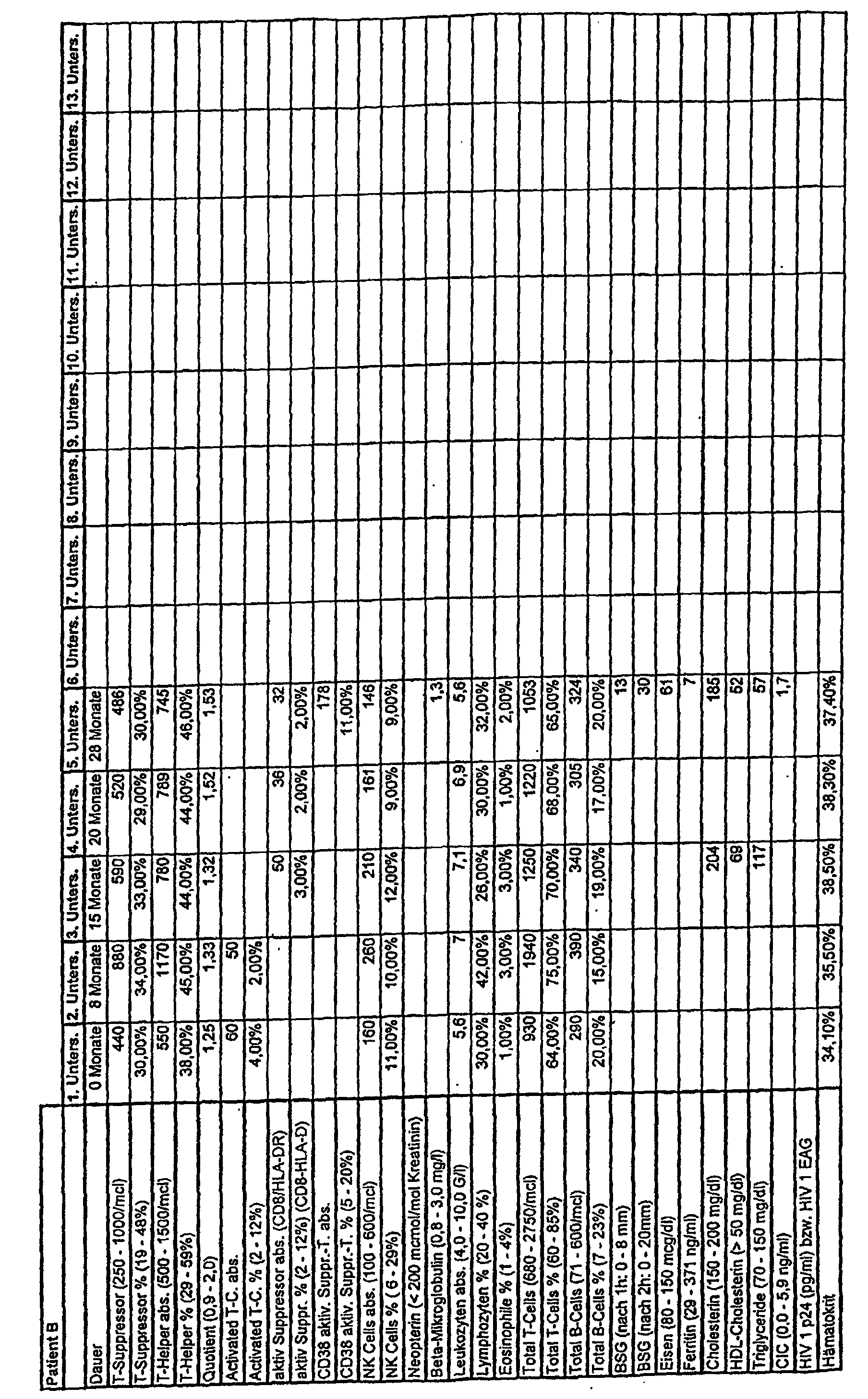

### Patientin C:

Während des Zeitraumes zwischen 1. und 3. Untersuchung ernährte sich die Patientin vollwertig und supplementierte Mikronährstoffe; zwischen der 3. und 4. Untersuchung ernährte sich die Patientin C vollwertig und supplementierte Mikronährstoffe in Kombination mit Vollwertkonzentraten. Die Ergebnisse bezüglich T-Helfer- und T-Suppressorzellen sind in Tabelle 5 dargestellt (Gesamtanalyse: Tabelle 6).

**TABELLE 5**

| Patientin C HIV-pos., weiblich | 1. Untersuchung | 3. Untersuchung 7 Monate | 4. Untersuchung 11 Monate |
|---|---|---|---|
| T-Helfer | 574 | 465 | 577 |
| T-Suppressor | 714 | 509 | 507 |
| Ratio | 0,80 | 0,91 | 1,14 |

Der Anstieg bzw. Abfall von T-Helferzellen bzw. der Verlauf der Ratio T-Helfer- zu T-Suppressorzellen gelten als wichtige Laborparameter zur Beurteilung des Immunsystemstatus von immunsupprimierten Patienten, insbesondere von HIV-positiven Patienten. Wie ein Vergleich der T-Helfer/T-Suppressorzellen-Werte, aber auch der übrigen Werte der drei angeführten Patienten zeigt, scheint eine Stabilisierung von Immunfunktionen am effektivsten durch die kombinierte Zufuhr standardisierter Mikronährstoffmengen auf Basis hochwertiger Vollwertkonzentrate erzielbar, wie sie mit dem erfindungsgemäßen Kombinationspräparat zur Verfügung gestellt werden können.

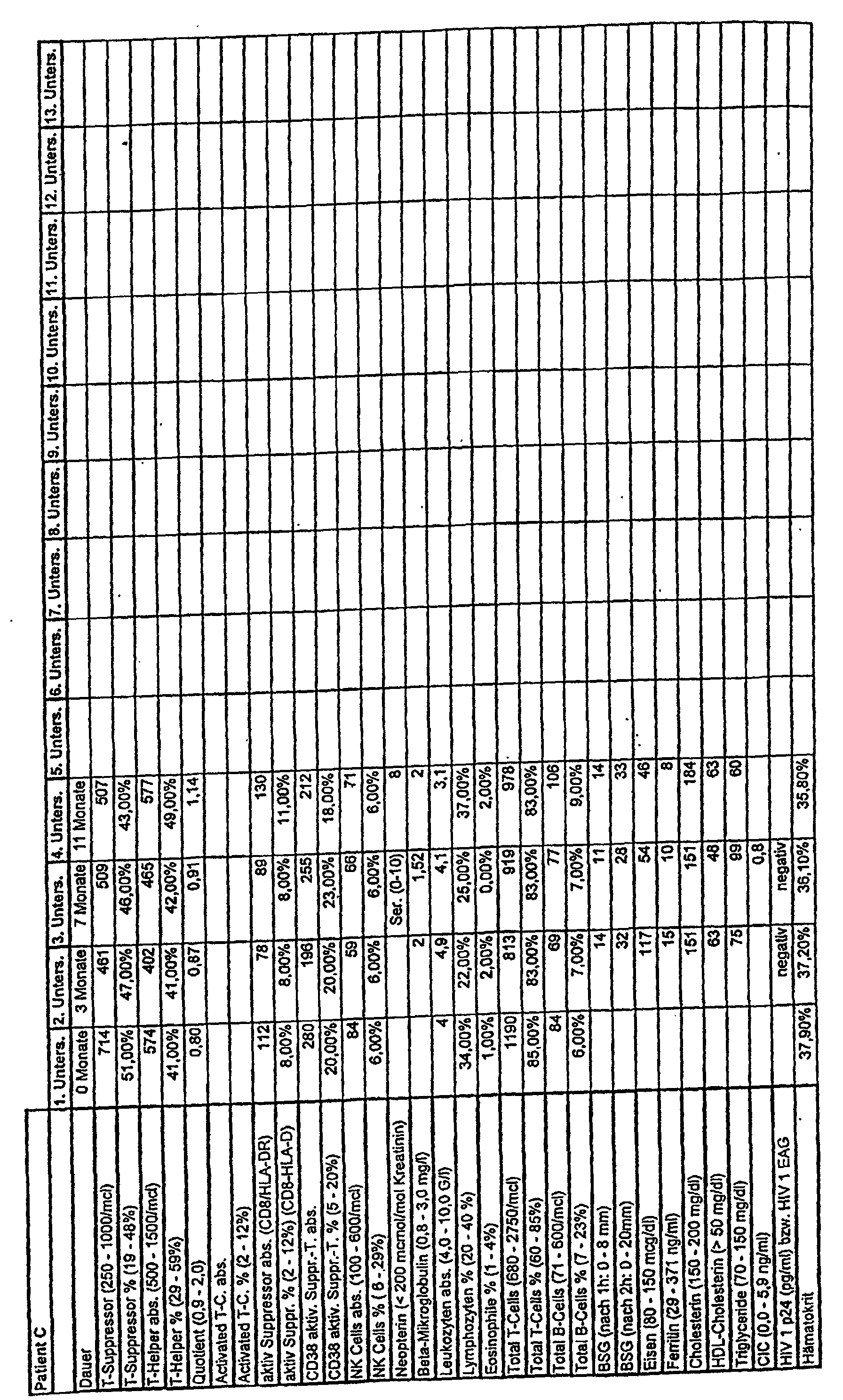

## Patentansprüche

1. Kombinationspräparat, umfassend 5-90% Elektrolyt-angereicherte, als Lebensmittel verwendbare Keimlinge, erhältlich durch Keimung in einer Elektrolytlösung, wobei die Elektrolytlösung 1 mg/l oder mehr Zn-, Fe-, K- und/oder Mg-Ionen, 0,5 mg/l oder mehr Cu-, Mn, Sr- und/oder Li-Ionen und 0,1 mg/l oder mehr Se-, Mo-, Cr-, As-, V- und/oder Co-Ionen enthält, und 5-90% zugesetzte essentielle, semiessentielle und/oder nicht essentielle Mikronährstoffe, ausgewählt aus der Gruppe bestehend aus: mehrfach ungesättigte Fettsäuren, natürlichen Carotenoidgemischen, Keimextrakten, natürlichen Anthocyangemischen, natürlichen Flavenoidgemischen, natürlichen Tocopherol- und Tocotrienolgemischen, Vitaminen und Coenzymen, essentiellen und nicht-essentiellen Aminosäuren, Mineralstoffen und Mischungen derselben.

2. Präparat nach Anspruch 1, **dadurch gekennzeichnet, dass** die Keimlinge Weizen-, Hafer- und/oder Buchenweizen-Keimlinge sind.

3. Präparat nach Anspruch 1 oder 2, **dadurch gekennzeichnet, dass** es zusätzlich ein oder mehrere Gemüsekonzentrate, vorzugsweise aus Broccoli, Petersilie und/oder Blumenkohl umfasst.

4. Präparat nach einem der Ansprüche 1 bis 3, **dadurch gekennzeichnet, dass** es 0 bis 90 % andere Komponenten, vorzugsweise Gemüsekonzentrate, lebensmitteltechnische Zusatzstoffe und/oder Hilfskomponenten, sowie vorzugsweise 20-70% Keimlinge und 20-70% Mikronährstoffe umfasst.

5. Präparat nach einem der Ansprüche 1 bis 4, **dadurch gekennzeichnet, dass** es in Form von Kapseln, Kautabletten, Pulvermi schung oder in einer lebensmitteltechnisch oder arzneimittel technisch geeigneten Form lagerstabil abgepackt ist.

6. Präparat nach einem der Ansprüche 1 bis 5, **dadurch gekennzeichnet, dass** es in einer oral zu verabreichenden pharmazeuti schen Darreichungsform vorliegt.

7. Präparat nach einem der Ansprüche 1 bis 6, **dadurch gekennzeichnet, dass** es als Zusatz mit herkömmlichen Lebensmitteln ver mischt ist.

8. Präparat nach einem der Ansprüche 1 bis 7, **dadurch gekennzeichnet, dass** die Mikronährstoffe ausgewählt sind aus der Gruppe bestehend aus
- mehrfach-ungesättigten Fettsäuren, vorzugsweise einem PUFA-Gemisch aus Fischöl und Borretschöl,
- natürlichen Carotenoidgemischen, vorzugsweise aus Dunaliella salina,
- Keimextrakten, vorzugsweise Weizenkeimextrakt mit Octacosanol,
- natürlichen Anthocyangemischen, vorzugsweise aus Hibiskusblüten,
- natürlichen Flavenoidgemischen, vorzugsweise aus Zitrusfrüchten, Baumharzen und Rinderextrakten,
- natürlichen Tocopherol- und Tocotrienolgemischen, vorzugsweise aus Weizenkeimen,
- Vitaminen und Coenzymen, vorzugsweise Coenzym Q10, Pyridoxol, Riboflavin, Folsäure, Biotin, Vitamin K, Vitamin B 12, Vitamin D3, Carnitin, Betain und/oder Vitamin C,
- essentiellen und nicht essentiellen Aminosäuren, vorzugsweise N-Acetylcystein, Taurin, L-Glutamin, Isoleucin, Leucin, Lysin, Methionin, Phenylalanin, Threonin, Tryptophan, Valin, Histidin, Arginin und/oder Tyrosin,
- Mineralstoffen, vorzugsweise Kieselerde, Calcium, Vanadium, Mangan, Eisen, Kalium, Zink, Kupfer, Lithium, Fluor, Germanium, Strontium, Chrom, Molybdän, Selen und/oder Jod, und
- anderen Mikronährstoffen, vorzugsweise Quercetin, α-Liponsäure, Glutathion, Aneurin, Inosit, Orotsäure, Inosin und/oder p-Aminobenzoesäure,
- oder Mischungen derselben.

9. Präparat nach einem der Ansprüche 1 bis 8 als Arzneimittel, Diätetikum oder Nahrungsergänzung.

10. Verwendung eines Präparates nach einem der Ansprüche 1 bis 8 für die Herstellung eines Arzneimittels zur Behandlung von immunsupprimierten Personen, insbesondere zur Behandlung von HIV-positiven Patienten.

11. Verwendung nach Anspruch 10 zur Steigerung der Konzentration der T-Helferzellen im Blut bzw. zur Steigerung des Quotienten aus T-Helferzellen und T-Suppressorzellen.

12. Verwendung nach Anspruch 10 zur Senkung eines pathologisch erhöhten Mikroglobulin- bzw. Neopterin-Spiegels.

13. Nicht medizinische Verwendung eines Präparates nach einem der Ansprüche 1 bis 8 als Diätetikum oder Nahrungsergänzung.

## Claims

1. A combination preparation comprising 5-90% of electrolyte-enriched plant embryos usable as a foodstuff, obtainable by germination in an electrolyte solution, the electrolyte solution consisting of 1 mg/l or more of Zn, Fe, K and/or Mg ions, 0.5 mg/l or more of Cu, Mn, Sr and/or Li ions, and 0.1 mg/l or more of Se, Mo, Cr, As, V and/or Co ions, and 5-90% of admixed essential, semi-essential and/or non-essential micro-nutrients, selected from the group consisting of poly-unsaturated fatty acids, natural carotenoid mixtures, plant embryo extracts, natural anthocyano-mixtures, natural flavenoid mixtures, natural tocopherol and tocotrienol mixtures, vitamins and coenzymes, essential and non-essential amino acids, minerals, and combinations thereof.

2. A preparation according to claim 1, **characterised in that** the plant embryos are wheat, oat and/or buckwheat embryos.

3. A preparation according to claim 1 or 2, **characterised in that** it additionally comprises one or more vegetable concentrates, preferably from broccoli, parsley and/or cauliflower.

4. A preparation according to any one of claims 1 to 3, **characterised in that** it comprises 0 to 90% of other components, preferably vegetable concentrates, food-technological additives and/or auxiliary components, as well as preferably 20-70% of plant embryos and 20-70% of micro-nutrients.

5. A preparation according to any one of claims 1 to 4, **characterised in that** it is packed in the form of capsules, chewing tablets, powder mixtures or in a food-technologically suitable form or in a pharmaceutical-technologically suitable form so as to be storage-stable.

6. A preparation according to any one of claims 1 to 5, **characterised in that** it is provided in a pharmaceutically administrable form for oral administration.

7. A preparation according to any one of claims 1 to 6, **characterised in that** it is mixed as an additive with conventional foodstuffs.

8. A preparation according to any one of claim 1 to 7, **characterised in that** the micro-nutrients are selected from the group consisting of
- poly-unsaturated fatty acids, preferably a PUFA-mixture of fish oil and borage oil,
- natural carotenoid mixtures, preferably from dunaliella salina,
- plant embryo extracts, preferably wheat embryo extracts with octacosanole,
- natural anthocyano-mixtures, preferably from hibiscus blossoms,
- natural flavenoid mixtures, preferably from citrus fruit, tree resins and bark extracts,
- natural tocopherol and tocotrienol mixtures, preferably from wheat embryos,
- vitamins and coenzymes, preferably coenzyme Q10, pyridoxole, riboflavin, folic acid, biotin, vitamin K, vitamin B 12, vitamin D3, carnitine, betain and/or vitamin C,
- essential and non-essential amino acids, preferably N-acetyl cysteine, taurine, L-glutamine, isoleucine, leucine, lysine, methionine, phenyl alanine, threonine, tryptophane, valine, histidine, arginine and/or tyrosine,
- minerals, preferably silica, calcium, vanadium, manganese, iron, potassium, zinc, copper, lithium, fluorine, germanium, strontium, chromium, molybdenum, selenium and/or iodine, and
- other micro-nutrients, preferably quercetin, α-lipoic acid, glutathione, aneurin, inositol, orotic acid, inosine and/or p-amino benzoic acid,
- or combinations thereof.

9. A preparation according to any one of claims 1 to 8 as a medicament, a dietetic or a food supplement.

10. The use of a preparation according to any one of claims 1 to 8 for preparing a medicament for treating immune-suppressed persons, in particular for treating HIV positive patients.

11. The use according to claim 10 for increasing the concentration of T helper cells in the blood, or for increasing the quotient of T helper cells and T suppressor cells, respectively.

12. The use according to claim 10 for lowering a pathologically increased microglobulin or neopterine level.

13. The non-medical use of a preparation according to any one of claims 1 to 8 as a dietetic or as a food supplement.

## Revendications

1. Préparation contenant, en combinaison, de 5 à 90 % de germes enrichis par un électrolyte, utilisables en tant qu'aliments, obtenus par germination dans un électrolyte, l'électrolyte contenant 1 mg/l ou plus d'ions Zn, Fe, K et/ou Mg, 0,5 mg/l ou plus d'ions Cu, Mn, Sr et/ou Li et 0,1 mg/l ou plus d'ions Se, Mo, Cr, As, V et/ou Co, et, ajouté à ces germes, de 5 à 90 % de micronutriments essentiels, semi-essentiels et/ou non essentiels, sélectionnés dans le groupe consistant en : les acides gras polyinsaturés, les mélanges de caroténoïdes naturels, les extraits de germes, les mélanges d'anthocyanes naturels, les mélanges de flavonoïdes naturels, les mélanges de tocophérols et tocotriénols, les vitamines et les coenzymes, les aminoacides essentiels et non essentiels, les substances minérales et les mélanges de ceux-ci.

2. Préparation selon la revendication 1, **caractérisée en ce que** les germes sont des germes de blé, d'avoine et/ou de sarrasin.

3. Préparation selon la revendication 1 ou 2, **caractérisée en ce que** ladite préparation contient, en outre, un ou plusieurs concentrés de légumes, de préférence à base de brocoli, de persil et/ou de chou-fleur.

4. Préparation selon l'une des revendications 1 à 3, **caractérisée en ce que** ladite préparation contient 0 à 90 % d'autres composants, de préférence des concentrés de légumes, des additifs de la technique agroalimentaire et/ou des composants auxiliaires, ainsi que, de préférence, de 20 à 70 % de germes et de 20 à 70 % de micronutriments.

5. Préparation selon l'une des revendications 1 à 4, **caractérisée en ce que** ladite préparation se présente sous une forme stable au stockage, soit sous forme de capsules, de comprimés à mâcher, de mélange de poudres, soit sous une forme appropriée à la technique agroalimentaire ou à la technique pharmaceutique.

6. Préparation selon l'une des revendications 1 à 5, **caractérisée en ce que** ladite préparation se présente sous forme de préparation pharmaceutique pour administration orale.

7. Préparation selon l'une des revendications 1 à 6, **caractérisée en ce que** ladite préparation est mélangée à des aliments classiques à titre d'additif.

8. Préparation selon l'une des revendications 1 à 7, **caractérisée en ce que** les micronutriments sont sélectionnés dans le groupe constitué par :
- les acides gras polyinsaturés, de préférence un mélange (PUFA-Gemisch) d'huile de poissons et d'huile de bourrache,
- les extraits de caroténoïdes naturels, de préférence à base de Dunaliella salina,
- les extraits de germes, de préférence les extraits des germes de blé avec de l'octacosanol,
- les mélanges d'anthocyanes naturels, de préférence tirés de fleurs d'hibiscus,
- les mélanges de flavonoïdes naturels, de préférence tirés de fruits de citrus, de résine d'arbre et d'extraits de boeuf,
- les mélanges de tocophérols et tocotriénols, de préférence tirés de germes de blé,
- les vitamines et les coenzymes, de préférence la coenzyme Q10, le pyridoxal, la riboflavine, l'acide folique, la biotine, la vitamine K, la vitamine B12, la vitamine D3, la carnitine, la bétaïne et/ou la vitamine C,
- les aminoacides essentiels et non essentiels, de préférence la N-acétylcystéine, la taurine, le L-glutamine, l'isoleucine, la leucine, la lysine, la méthionine, la phénylalanine, la thréonine, le tryptophane, la valine, l'histidine, l'arginine et/ou la tyrosine,
- les substances minérales, de préférence le dioxyde de silicium, le calcium, le vanadium, le manganèse, le fer, le kalium, le zinc, le cuivre, le lithium, le fluor, le germanium, le strontium, le chrome, le molybdène, le sélénium et/ou l'iode, et
- d'autres micronutriments, de préférence, la quercétine, l'acide α-lipoïque, le glutathion, l'aneurine, l'inosite, l'acide orotique, l'inosine et/ou l'acide p-aminobenzoïque,
- ou des mélanges de ceux-ci.

9. Préparation selon l'une des revendications 1 à 8 sous forme de produit pharmaceutique, de produit diététique ou de complément alimentaire.

10. Utilisation d'une préparation selon l'une des revendications 1 à 8 pour la fabrication d'un produit pharmaceutique destiné au traitement des sujets immunodéficitaires, en particulier pour le traitement des patients séropositifs au virus VIH.

11. Utilisation selon la revendication 10, soit pour augmenter la concentration des cellules T régulatrices dans le sang, soit pour augmenter le rapport des cellules T régulatrices aux cellules T suppressives.

12. Utilisation selon la revendication 10, pour diminuer un taux pathologiquement élevé de microglobuline ou de néoptérine.

13. Utilisation non médicale d'une préparation selon l'une des revendications 1 à 8 sous forme de produit diététique ou de complément alimentaire.
